# EUROPÄISCHE PATENTANMELDUNG

(11) **EP 1 837 007 A2**
(43) Veröffentlichungstag der Anmeldung: **26.09.2007**
(21) Anmeldenummer: 07104083.6
(22) Anmeldetag: 14.03.2007
(51) Int. Cl.: A61K 8/04, A61K 8/29, A61K 8/37, A61K 8/49, A61K 8/81, A61Q 17/04

(54) **Sprühbare Sonnenschutzlotion**

(30) Priorität: 20.03.2006 DE 102006013283
(71) Anmelder: Beiersdorf AG, 20245 Hamburg (DE)
(72) Erfinder: Mundt, Claudia, 28207, Bremen (DE); Skubsch, Kerstin, 25421, Pinneberg (DE); Windisch, Björna, 22607, Hamburg (DE); Weingarz, Yvonne, 22763, Hamburg (DE)

(57) **Zusammenfassung**

Sprühbare Sonnenschutzlotion enthaltend
a) 5 bis 40 Gewichts-% Lipide,
b) 40 bis 80 Gewichts-% Wasser,
c) 0,1 bis 2 Gewichts-% Verdickungsmittel,
d) 0,1 bis 5 Gewichts-% hydrophil beschichtete Titandioxid-Partikel,
e) Neutralisationsmittel,

wobei sich die Gewichtsangaben auf das Gesamtgewicht der Zubereitung beziehen, dadurch gekennzeichnet, dass die Zubereitung einen pH-Wert von 5,5 bis 7,8 aufweist.

## Beschreibung

Die vorliegende Erfindung betrifft eine sprühbare Sonnenschutzlotion.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Dem Verbraucher werden Sonnenschutzmittel in der Regel in Form von Lotionen oder in Form von Sprays angeboten.

Sonnenschutzsprays haben den Vorteil, dass sie einfach und schnell aufzutragen sind. Durch die Vernebelung können größere Körperpartien mit Sonnenschutz bedeckt werden. Spray sind sehr praktisch in der Anwendung. Meistens werden sie außerdem von dem Verbraucher als wenig fettig und klebrig empfunden.

Die Sprays haben jedoch den Nachteil, dass der Sprühnebel auch außerhalb des Körpers auf z.B. Handtuch, Fliesen etc. verteilt wird. Außerdem sind sie schwierig im Gesicht anzuwenden, weil eventuell Sprühnebel in die Augen dringt. Deswegen verwenden die meisten Verbraucher das Spray so, dass sie es sich erst in eine Hand sprühen und die Formulierung dann im Gesicht verteilen. Meistens sind die Sprays allerdings so dünnflüssig, dass es passieren kann, dass die Flüssigkeit aus der Hand rinnt.

Es gibt jedoch auch Verbraucher, die Sonnenschutzlotionen bevorzugen. Sie haben den Vorteil, dass man durch die höhere Konsistenz eine größere Menge auftragen kann. Dadurch erhöht sich die Schutz- und Pflegeleistung. Besonders bei trockener Haut werden Lotionen bevorzugt. Lotionen sind außerdem auch gut im Gesicht anwendbar.
Sonnenschutzlotionen haben den Nachteil, dass die Verteilung relativ zeitaufwendig ist. Von manchen Verbrauchern werden sie außerdem als etwas zähflüssig und fettig empfunden.

Darüber hinaus ist es für die Hersteller von Sonnenschutzmitteln von Nachteil, dass sie für jeden Zubereitungstyp (Spray, Lotion) eine eigene Rezeptur zu entwickeln müssen.

Es war daher die Aufgabe der vorliegenden Erfindung, eine Sonnenschutzzubereitung zu entwickeln, die sowohl als Lotion als auch als Spray einsetzbar ist und die Vorteile beider Zubereitungsformen in sich vereinigt.

Überraschend gelöst werden die Aufgaben durch eine sprühbare Sonnenschutzlotion enthaltend
a) 5 bis 40 Gewichts-% Lipide,
b) 40 bis 80 Gewichts-% Wasser
c) 0,1 bis 2 Gewichts-% Verdickungsmittel,
d) 0,1 bis 5 Gewichts-% hydrophil beschichtete Titandioxid-Partikel,
e) Neutralisationsmittel,
wobei sich die Gewichtsangaben auf das Gesamtgewicht der Zubereitung beziehen, dadurch gekennzeichnet, dass die Zubereitung einen pH-Wert von 5,5 bis 7,8 aufweist.

Die erfindungsgemäßen Zubereitungen zeigen ein schönes Sprühbild mit gleichmäßigen, feinen Tröpfchen und sind als Lotion gut dosierbar. Nachdem Versprühen sind sie jedoch so zähflüssig, dass sie selbst auf senkrecht aufgerichteten Hautpartien nur langsam zerfließen. Das rheologische Verhalten sorgt darüber hinaus dafür, dass die Zubereitung sprühbar (d.h. unter den Förderbedingungen eines Sprühapplikators hinreichend dünnflüssig ist) andererseits, im "nicht-sprühenden" Zustand so zähflüssig ist, dass keinerlei Gefahr besteht, dass sie aus einem herkömmlichen Packmittel für Lotionen "auslaufen" kann, wie es bei "normaldünnflüssigen" Formulierungen der Fall ist. Die Zubereitungen lassen sich leicht auf der Haut verteilen. Die Wasserfestigkeit der erfindungsgemäßen Zubereitungen ist überraschend hoch. Obwohl hydrophil beschichtete Titandioxid-Partikel in der Zubereitung enthalten sind, bleibt der UV-Schutz der Haut durch die Zubereitung auch nach dem Wasserkontakt (beispielsweise nach dem Baden) ungewöhnlich gut erhalten. Die erfindungsgemäßen Zubereitungen lassen sich sowohl als Spray als auch als "normale" Lotion verwenden.

Zwar kennt der Stand der Technik die DE 103 30 029, WO 00/66076, DE 19938756 und DE 19834819, WO 01/12146 doch konnten diese Schriften nicht den Weg zur vorliegenden Erfindung weisen.

Erfindungsgemäß wird unter einer Lotion eine Zubereitung verstanden, die aus einer opaken, meist weissen, zähflüssigen Öl-in-Wasser-Emulsion besteht mit einer Viskosität von ca. 2000 bis 7000mPas (gemessen mit Haake VT 02 Viscotester bei 25°C) und gut aus einer Lotionflasche mit z.B. Flip-Top-Verschluss entnehmbar ist.

Erfindungsgemäß wird unter einer sprühbaren Zubereitung eine Zubereitung verstanden, die aus einer Flüssigkeit besteht, welche sich mit Hilfe eines für kosmetische Sonnenschutzzubereitungen handelsüblichen Sprühkopfes gut versprühen lässt und dabei einen feinen Sprühnebel erzeugt (Lotionen hingegen erzeugen, falls sie überhaupt mit einem Sprühapplikator förderbar sind, ein "Sprühbild" aus groben, heterogen verteilten "Klecksen". In der Regel führt der Einsatz von nicht erfindungsgemäßen Titandioxid-Partikeln in solchen Zubereitungen des Standes der Technik schnell zur Verstopfung des Sprühapplikators). Als Sprühkopf kann z.B. der Pumpzerstäuber von der Firma Seaquest Perfect Dispensing GmBH mit der Bezeichnung PZ2 verwendet werden.

Erfindungsgemäß als "hydrophil" gelten, im Gegensatz zu hydrophoben oder amphiphilen Titandioxid-Partikeln, diejenigen Titandioxid-Partikel, welche so beschichtet sind, dass eine gute Einarbeitung, leichte Dispergierbarkeit und Absetzstabilität in wässrigen Phasen vorliegt.

Die Beschichtung kann aus ein oder mehreren Stoffen bestehen. Diese Beschichtung kann sowohl aus anorganischem als auch aus organischem Material bestehen.
Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9). Diese anorganischen Oberflächenbeschichtungen können allein, in Kombination und/oder in Kombination mit organischen Beschichtungsmaterialien vorkommen.

Im Sinne der vorliegenden Erfindung sind besonders bevorzugte Titandioxide das MT-500SA , MT 500 SAS und MT-100 AQ von Tayca Corporation, Eusolex T-AVO von Merck, Tioveil AQ 10 N von Uniquema und das Mirasun TiW60 von Rhone-Poulenc.

Es ist erfindungsgemäß vorteilhaft, wenn die erfindungsgemäßen Titandioxid-Partikel mit Siliciumdioxid beschichtet sind.

Bei erfindungsgemäß vorteilhaften Ausführungsformen der vorliegenden Erfindung beträgt der durchschnittliche Partikeldurchmesser der erfindungsgemäßen Titandioxid-Partikel von 5 bis 100 nm.

Es ist erfindungsgemäß bevorzugt, wenn die erfindungsgemäße Zubereitung Lipide in einer Gesamtkonzentration von 5 bis 20 Gewichts-%, bezogen auf das Gesamtgewicht der Zubereitung enthalten.

Überraschenderweise konnte mit Hilfe des hydrophil beschichtetem Titandioxid die Wasserfestigkeitsleistung des Produktes gesteigert werden. Die Untersuchung erfolgte nach der SPF Test Method (ctfa-Colipa-JCIA 2003) und Guideline for Evaluating Sun Product Water Resistance (Colipa 2004).

Es ist erfindungsgemäß bevorzugt, wenn die erfindungsgemäße Zubereitung Wasser in einer Gesamtkonzentration von 50 bis 70 Gewichts-%, bezogen auf das Gesamtgewicht der Zubereitung enthält.

Es ist erfindungsgemäß bevorzugt, wenn die erfindungsgemäße Zubereitung Verdickungsmittel in einer Gesamtkonzentration von 0,3 bis 1 Gewichts-%, bezogen auf das Gesamtgewicht der Zubereitung enthalten.

Es ist erfindungsgemäß bevorzugt, wenn die erfindungsgemäße Zubereitung Titandioxid-Partikel in einer Gesamtkonzentration von 0,5 bis 2 Gewichts-%, bezogen auf das Gesamtgewicht der Zubereitung enthalten.

Es ist erfindungsgemäß bevorzugt, wenn die erfindungsgemäße Zubereitung einen pH-Wert von 5,5 bis 7,8 aufweist.

Erfindungsgemäß vorteilhafte Ausführungsformen der erfindungsgemäßen Sonnenschutzlotion sind dadurch gekennzeichnet, dass die Zubereitung eine Fließgrenze von 5 bis 30 Pa aufweist. Dabei ist eine Fließgrenze von 7 bis 25 Pa erfindungsgemäß bevorzugt.

Die erfindungsgemäße Fließgrenze wird dabei wie folgt definiert:
Die kritische Schubspannung der Fließkurve.
Sie kann erfindungsgemäß wie folgt ermittelt werden:
Gemessen wird die Fließkurve auf einem schubspannungsgesteuerten Rheometer bei 25°C +/-1°C mit 25 mm Platte/Platte Geomatrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Es ist erfindungsgemäß von Vorteil, wenn die erfindungsgemäße Sonnenschutzlotion einen Lichtschutzfaktor von größer oder gleich 5 aufweist. Dabei ist ein Lichtschutzfaktor von größer oder gleich 10 erfindungsgemäß bevorzugt. Der erfindungsgemäße Lichtschutzfaktor kann dabei nach den Vorschriften der New International SPF Method (Februar 2003) in vivo auf menschlicher Haut bestimmt werden.

Für die erfindungsgemäße Sonnenschutzlotion ist es erfindungsgemäß vorteilhaft, wenn die Verdickungsmittel gewählt werden aus der Gruppe der Verbindungen Siliciumdioxid, Schichtsilikate, Polysaccharide bzw. deren Derivate, z. B. Alginate, Gellan Gum, Carrageenate, Pectin, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2020, 2984, 5984 und Permulen TR1 ,TR 2, jeweils einzeln oder in Kombination.
Erfindungsgemäß bevorzugt ist es ein oder mehrere Verdickungsmittel gewählt aus der Gruppe der vernetzten Polyacrylatsäuren (INCI: Acrylates/C10-30 Alkyl Acrylate Crosspolymere), z.B. die Substanzen Carbopol 1328, Permulen TR1 und TR2 der Firma Noveon, einzusetzen.

Es ist für die erfindungsgemäße Sonnenschutzlotion erfindungsgemäß vorteilhaft, wenn der Emulgator-Gehalt der Zubereitung kleiner oder gleich 0,5 Gewichts-%, bezogen auf das Gesamtgewicht der Zubereitung, beträgt. Erfindungsgemäß bevorzugt sind dabei emulgatorfreie Zubereitungen (0 Gew.-% Emulgator).

Es ist vorteilhaft im Sinne der vorliegenden Erfindung, wenn als Neutralisationsmittel ein oder mehrere Verbindungen gewählt aus der Gruppe Natriumhydroxid, Kaliumhydroxid,Ammoniumhydroxid,Arginin,Trometamol, Triethanolamin eingesetzt werden. Dabei ist der Einsatz von Natronlauge erfindungsgemäß bevorzugt.

Die erfindungsgemäße Sonnenschutzlotion kann erfindungsgemäß vorteilhaft einen oder mehrere UV-Lichtschutzfilter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine, 2,4,6-Tribiphenyl-4-yl-1,3,5-triazin; Zinkoxid in einer Konzentration von 0,01 bis 40 Gewichts-%, bezogen auf das Gesamtgewicht der Zubereitung, enthalten.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-% bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Hydroxyethylharnstoff, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner.

Erfindungsgemäß vorteilhaft enthält die erfindungsgemäße Zubereitung einen oder mehrere Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP) 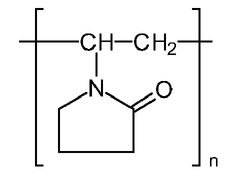 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole. Ebenfall vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann sowie Dermacryl 79 (Acrylates/Octylacrylamide Copolymer) von National Starch.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenylethylbenzoat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon *(Hallstar AB)* und/oder Diethylhexylnaphthalat *(Hallbrite TQ oder Corapan TQ von H&R).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, dass die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Erfindungsgemäß ist auch das Verfahren zur Herstellung der erfindungsgemäßen Sonnenschutzlotion. Dieses Herstellungsverfahren ist dadurch gekennzeichnet, dass das hydrophile Titandioxid in die geschmolzene Fettphase gegeben wird. Außerdem ist es wichtig, dass der Verdicker in die Wasserphase eindispergiert wird und erst nach dem Homogenisieren und Kaltrühren mit z.B. Natronlauge neutralisiert wird.

Erfindungsgemäß ist auch die Zubereitung hergestellt nach dem erfindungsgemäßen Herstellungsverfahren.

Erfindungsgemäß ist nicht zuletzt ein Packmittel enthaltend eine erfindungsgemäße Sonnenschutzlotion, dadurch gekennzeichnet, dass die Zubereitung aus dem Packmittel sowohl durch eine Lochöffnung als auch mit Hilfe eines Sprühkopfes mit mechanischer Förderpumpe entnommen werden kann.

Beispiele für diese Packmittel sind in der deutschen Patentanmeldung mit dem Aktenzeichen 10 2005 028 364.0 beschrieben.

Erfindungsgemäß ist die Verwendung hydrophil beschichteter Titandioxid-Partikel zur Erhöhung der Viskosität sprühbarer Sonnenschutzzubereitungen und die Verwendung hydrophil beschichteter Titandioxid-Partikel zur Herstellung wasserfester Sonnenschutzzubereitungen.

### Vergleichsversuch

Der überraschende Vorteil, welcher beim Einsatz der erfindungsgemäßen Titandioxid-Partikel im Gegensatz zu anderen Titandioxid-Partikeln zu beobachten ist, wird beispielsweise aus den folgenden Vergleichsversuchen ersichtlich:

| | | | **A** | **B** | **C** | **D** | **E** |
|---|---|---|---|---|---|---|---|
| **Handelsname** | | | **m [%]** | **m [%]** | **m [%]** | **m [%]** | **m [%]** |
| Eusolex-T-AVO | Titandioxid + Silica (beschichtet) | hydrophil | | 1,0 | | | |
| Tego Sun T 805 | Titandioxid +Trimethoxycaprylylsilan | hydrophob | | | 1,0 | | |
| Eusolex-T-2000 | Titanium Dioxide + Alumina + Simethicone | hydrophob | | | | 1,0 | |
| Tioveil AQ N | Titanium Dioxide + Alumina + Silica + Sodium Polyacrylate | hydrophil | | | | | 1,0 |
| Carbopol 1382 | Acrylates/C10-30 Alkyl Acrylate Crosspolymer | | 0,3 | 0,3 | 0,3 | 0,3 | 0,3 |
| | Alkohol | | 4,0 | 4,0 | 4,0 | 4,0 | 4,0 |
| | Bis-Ethylhexyloxyphenol Methoxyphenyl Triazin | | 1,0 | 1,0 | 1,0 | 1,0 | 1,0 |
| | Butyl Methoxydibenzoylmethan | | 4,5 | 4,5 | 4,5 | 4,5 | 4,5 |
| | Butylene Glycol Dicaprylate/Dicaprat | | 6,0 | 6,0 | 6,0 | 6,0 | 6,0 |
| | C18-36 Acid Triglycerid | | 1,0 | 1,0 | 1,0 | 1,0 | 1,0 |
| | Ethylhexyl Methoxycinnamat | | 7,0 | 7,0 | 7,0 | 7,0 | 7,0 |
| | VP/Hexadecene Copolymer | | 0,5 | 0,5 | 0,5 | 0,5 | 0,5 |
| | Glycerin | | 5,0 | 5,0 | 5,0 | 5,0 | 5,0 |
| | Tocopheryl Acetat | | 0,5 | 0,5 | 0,5 | 0,5 | 0,5 |
| | Trisodium EDTA | | 1,0 | 1,0 | 1,0 | 1,0 | 1,0 |
| | Konservierungsmittel | | q.s. | q.s. | q.s. | q.s. | q.s. |
| | Parfüm | | q.s. | q.s. | q.s. | q.s. | q.s. |
| | Natriumhydroxid | | q.s. | q.s. | q.s. | q.s. | q.s. |
| | Wasser | | ad 100 | ad 100 | ad 100 | ad 100 | ad 100 |
| | Viskosität (mPa*s) (Haake Viskotester VT-02, 25°C) | | 1800 | 3500 | 1100 | 750 | 2900 |
| | Wasserfestigkeit | | 57% | 68% | | | |

| | | | | | | | |
|---|---|---|---|---|---|---|---|
| Die Muster Beispiel B und E sind trotz der recht hohen Viskosität von 3500 mPas bzw. 2900 mPas sprühbar. Beispiel B besitzt eine Fliessgrenze von 23 Pa (kritsche Schubspannung). | | | | | | | |

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

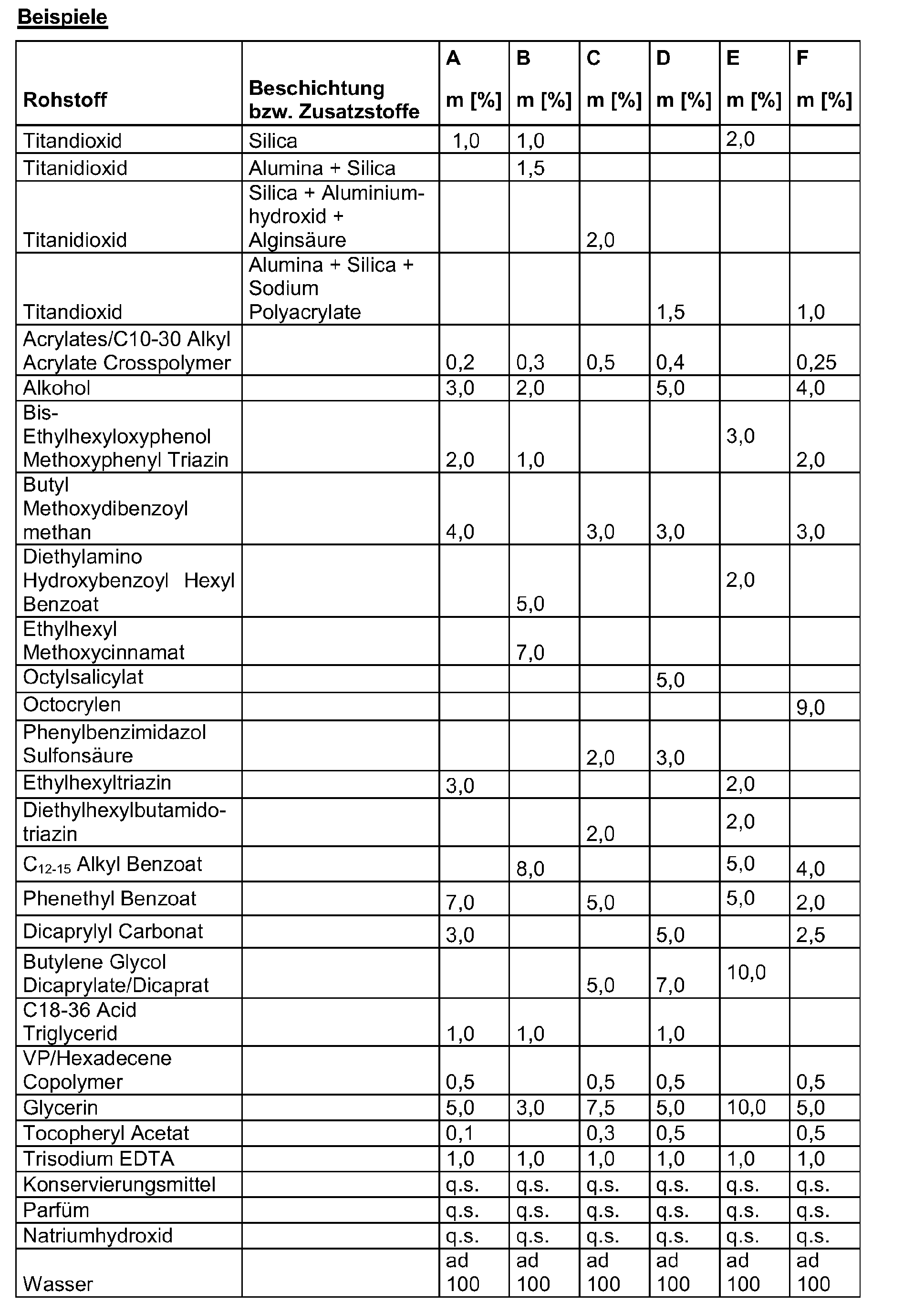

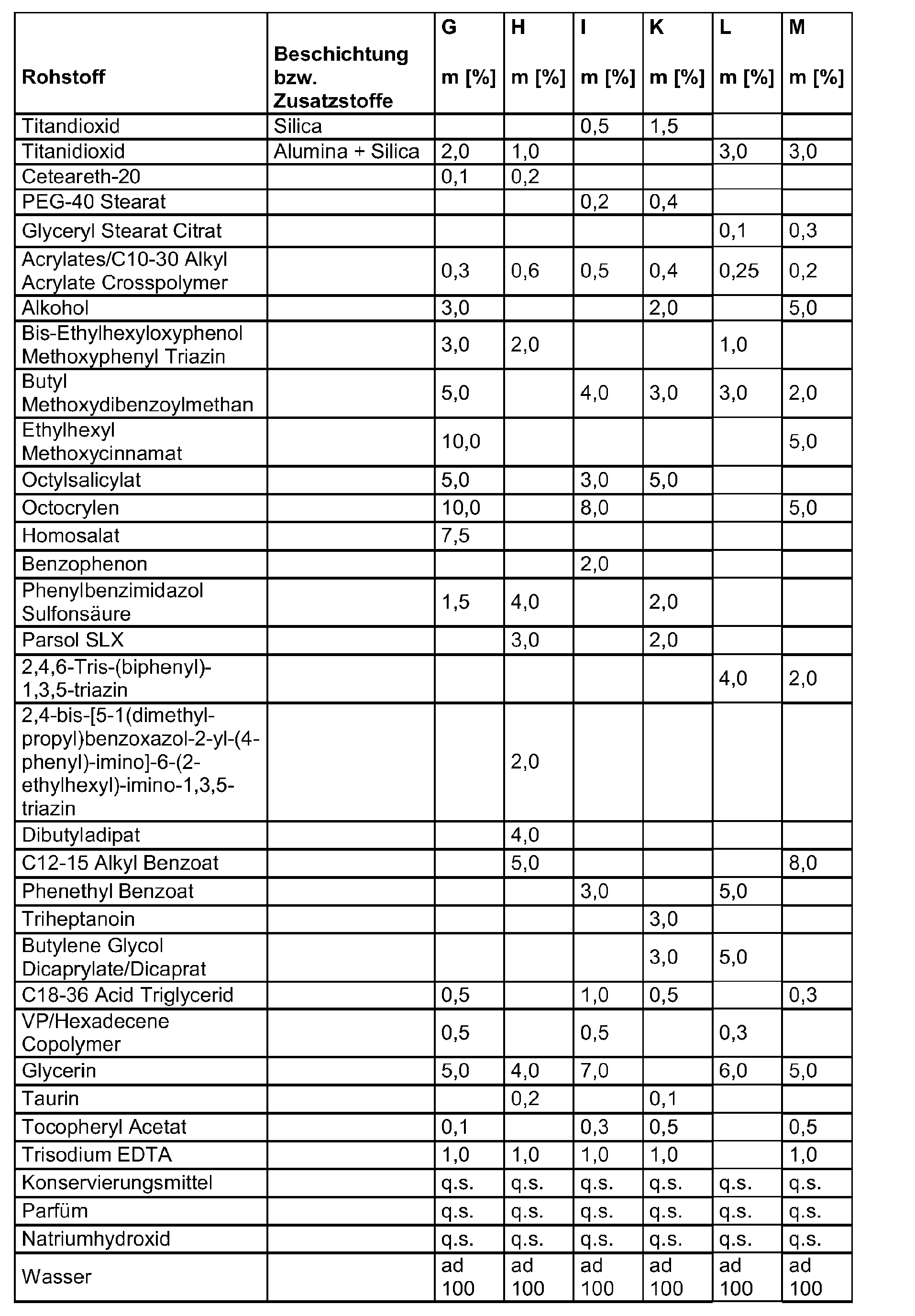

## Patentansprüche

1. Sprühbare Sonnenschutzlotion enthaltend
a) 5 bis 40 Gewichts-% Lipide,
b) 40 bis 80 Gewichts-% Wasser,
c) 0,1 bis 2 Gewichts-% Verdickungsmittel,
d) 0,1 bis 5 Gewichts-% hydrophil beschichtete Titandioxid-Partikel,
e) Neutralisationsmittel,
wobei sich die Gewichtsangaben auf das Gesamtgewicht der Zubereitung beziehen, **dadurch gekennzeichnet, dass** die Zubereitung einen pH-Wert von 5,5 bis 7,8 aufweist.

2. Sonnenschutzlotion nach Anspruch 1, **dadurch gekennzeichnet, dass** die Zubereitung eine Fließgrenze von 5 bis 30 Pa aufweist.

3. Sonnenschutzlotion nach einem der vorhergehenden Ansprüche, **dadurch gekennzeichnet, dass** die Zubereitung einen Lichtschutzfaktor von größer oder gleich 5 aufweist.

4. Sonnenschutzlotion nach einem der vorhergehenden Ansprüche, **dadurch gekennzeichnet, dass** die Verdickungsmittel gewählt werden aus der Gruppe der vernetzten Polyacrylsäuren (INCI:Acrylates/C10-30 Alkyl Acrylate Crosspolymere)

5. Sonnenschutzlotion nach einem der vorhergehenden Ansprüche, **dadurch gekennzeichnet, dass** der Emulgator-Gehalt der Zubereitung kleiner oder gleich 0,5 Gewichts-%, bezogen auf das Gesamtgewicht der Zubereitung, beträgt.

6. Sonnenschutzlotion nach einem der vorhergehenden Ansprüche, **dadurch gekennzeichnet, dass** die Titandioxid-Partikel mit Siliciumoxid beschichtet sind.

7. Sonnenschutzlotion nach einem der vorhergehenden Ansprüche, **dadurch gekennzeichnet, dass** als Neutralisationsmittel ein oder mehrere Verbindungen gewählt aus der Gruppe Natriumhydroxid, Kaliumhydroxid,Ammoniumhydroxid,Arginin,Trometamol, Triethanolamin eingesetzt werden.

8. Sonnenschutzlotion nach einem der vorhergehenden Ansprüche, **dadurch gekennzeichnet, dass** sie einen oder mehrere UV-Lichtschutzfilter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 2-Methyl-5-(2-oxo-3-bornylidenmethyl)-sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyl-oxy)]-1,3,5-triazin (INCl: Ethylhexyl Triazone); Merocyanine, 2,4,6-Tribiphenyl-4-yl-1,3,5-triazin; Zinkoxid in einer Konzentration von 0,01 bis 40 Gewichts-%, bezogen auf das Gesamtgewicht der Zubereitung, enthält.

9. Verfahren zur Herstellung einer Sonnenschutzlotion nach einem der vorhergehenden Ansprüche, **dadurch gekennzeichnet, dass** das hydrophile Titandioxid in die geschmolzene Fettphase gegeben wird, der Verdicker in die Wasserphase eindispergiert wird und erst nach dem Homogenisieren und Kaltrühren neutralisiert wird.

10. Zubereitung hergestellt nach einem Verfahren nach Anspruch 9.

11. Packmittel enthaltend eine Sonnenschutzlotion nach einem der vorhergehenden Ansprüche, **dadurch gekennzeichnet, dass** die Zubereitung aus dem Packmittel sowohl durch eine Lochöffnung als auch mit Hilfe eines Sprühkopfes mit mechanischer Förderpumpe entnommen werden kann.
